Europäisches Patentamt

European Patent Office

Office européen des brevets

(11) Publication number: **0 680 763 A2**

## EUROPEAN PATENT APPLICATION

(21) Application number: **95106815.4**

(22) Date of filing: **05.05.95**

(51) Int. Cl.6: **A61K 38/48**, C12N 9/96,
//(A61K38/48,38:36)

(30) Priority: **06.05.94 DE 4416180**

(43) Date of publication of application:
**08.11.95 Bulletin 95/45**

(84) Designated Contracting States:
**AT BE CH DE DK ES FR GB IT LI NL SE**

(71) Applicant: **IMMUNO AG**
**Industriestrasse 67**
**A-1221 Wien (AT)**

(72) Inventor: **Eibl, Johann, Dr.**
**Gustav Tschermak-G. 2**
**A-1180 Vienna (AT)**
Inventor: **Schwarz, Hans Peter, Doz.**
**Schindlergasse 32**
**A-1180 Vienna (AT)**
Inventor: **Siekmann, Jürgen, Dr.**
**Gerasdorfer Strasse 153/209**
**A-1210 Vienna (AT)**
Inventor: **Turecek, Peter, Dr.**
**Hutweidengasse 41**
**A-1190 Vienna (AT)**

(74) Representative: **Kolb, Helga, Dr. Dipl.-Chem. et al**
**Hoffmann, Eitle & Partner,**
**Patent-und Rechtsanwälte,**
**Arabellastrasse 4**
**D-81925 München (DE)**

(54) **Stable preparation for the treatment of blood coagulation disorders comprising an activated coagulation factor and lipid vesicles.**

(57) The invention relates to a stable preparation for the treatment of blood coagulation disorders which comprises an active coagulation promoting substance, such as for example an activated coagulation factor and optionally other proteins, which are bound in or on lipid vesicles. The preparations can can be made available in a virus inactivated form.

The invention also relates to the use of lipid vesicles which are bound with thrombocyte-like blood coagulation factors for the production of a preparation for the treatment of blood coagulation disorders which are connected with a lack of activated thrombocytes.

Further, the invention relates to methods for the production of preparations for the treatment of blood coagulation disorders.

EP 0 680 763 A2

The invention relates to a stable preparation for the prevention and treatment of blood coagulation disorders which comprises an active coagulation promoting substance, such as for example an activated coagulation factor and optionally other proteins.

Coagulation disorders of blood occur when for example the coagulation time deviates from the standard value due to the lack of a coagulation component. The disorders can be inherited as well as acquired through disease conditions.

Hemophilia A is one of the most frequently occurring inherited coagulation disorders. Patients with hemophilia A are prone to frequent hemorrhages as a result of a shortage of Factor VIII which can be treated with Factor VIII concentrates. However, in about 15% of the patients the occurrence of Factor VIII neutralizing antibodies, so-called inhibitors, results whereby a therapy with Factor VIII concentrates is hardly possible.

For the treatment of hemophilia A inhibitor patients, complex mixtures of coagulation factors have proven themselves. The activated prothrombin complex FEIBA® (Immuno) has a "factor eight inhibitor bypassing activity".

In lieu of Factor VIII concentrates, it was also attempted to treat hemophilia A with a mixture of coagulation factor Xa and phospholipids. In US 4,610,880, the treatment of hemophilic dogs with a mixture of Factor Xa and phospholipid vesicles is described. This mixture was not stable, for which reason a Factor Xa-containing solution and a suspension of phospholipid vesicles had to be freshly mixed immediately before use. It is stressed that the mixture ratio must be selected in such a way that hemostasis is achieved but thromboses are not caused.

It is known that phospholipid vesicles in combination with Factor Xa increase the danger of thrombosis On the basis of an in vivo stasis model with rabbits in Blood 59, 401-407 (1982), the increased thrombogenicity of Factor Xa is described when this was tested together with synthetic phospholipids (phosphatidyl choline phosphatidyl serine lipid vesicles, PCPS vesicles). The danger of thrombosis after administration of prothrombin complex concentrates is also attributed to the combination of coagulation active phospholipids and activated coagulation factors.

It is known from US 4,348,384 to incorporate the coagulation factors Factor VIII and IX in liposomes. Therewith, a preparation which can be orally or intestinally administered for the treatment of hemophilia A or B is made available. Here, the liposomes have a size of $1\mu$m and protect the Factor VIII or Factor IX from a premature digestion. The administration of these preparations holds no danger of thrombosis because it does not occur intravenously.

The instability of activated coagulation factors in storage is known. A method is known from Thrombosis Research 22, 213-220 (1981) which permits an as stable as possible beta-Factor Xa preparation to be obtained. According to this method, beta-Factor Xa is stored refrigerated in 50% glycerol at pH 7.2 in 0.03 M imidazol buffer. However, a further conversion to inactive fragments also occurs by use of this method even at 4°C. In a similar manner, the instability of Factor Xa in stabilizing glycerol-water mixtures is also described in J. Biol. Chem. 248, 7729-7741 (1973).

The object of the invention is to make available a stable preparation for the treatment of blood coagulation disorders comprising an active coagulation-promoting substance alone or in combination with coagulation factors and/or cofactors or fibrinolysis factors. An "active coagulation-promoting substance" is defined as a substance which demonstrates a coagulation-promoting activity without previous activation. As examples, all activated coagulation factors and cofactors can be selected.

A further object of the invention is to make available a stabile preparation for the treatment of blood coagulation disorders which comprises an active coagulation-promoting substance and was subjected to a treatment for the inactivation of potentially existing viruses.

The above named objects are solved according to the invention through a stabile preparation comprising a complex of suitable active coagulation-promoting substances and lipids in vesicular form. It has been demonstrated that the stability of an activated coagulation factor is increased in an unexpected manner through the association of an activated coagulation factor such as Factor Xa on a phospholipid in vesicular form, i.e. through the binding in and on a lipid vesicle. Not only an exceptionally good storage stability in solution or frozen and/or lyophilized from is achieved therewith. It can even be demonstrated that an activated coagulation factor is protected from physical inactivation such as for example in lyophilization or in a treatment for virus inactivation. The in vivo activity of the activated coagulation factor is also maintained, which is also a measure for the stability of the preparation.

A preparation according to the invention is considered as stable when the activity of the active coagulation-promoting substance is retained to more than 40%, preferably more than 50% through measures such as the lyophilization and reconstitution in the absence of customary stabilizers in unbuffered solution and/or when more than 40% of the activity of the active coagulation-promoting substance,

2

preferably more than 50%, most preferably more than 60%, is retained by the storage of an aqueous solution of the preparation in the absence of customary stabilizers at 22°C after 20 hours.

As determined by the exceptionally high stability, which was also established in vivo after infusion of the preparation according to the invention, the preparation is exceptionally suitable for the prevention and treatment of blood coagulation disorders.

Based on its structure, which is characterized by a protein and a lipid component, a complex of a thrombocytic coagulation active substance, i.e. for example a blood coagulation factor that is naturally present in thrombocytes, is suitable according to the invention as a thrombocyte substitute alone or in combination with further coagulation active substances and a lipid vesicle, and, therewith, for the prevention and treatment of blood coagulation disorders which are connected with a shortage of activated thrombocytes. For one thing, the complex according to the invention imitates the form of a blood platelet, for another functional similarities can be established, such as for example aggregation capability in a protein solution or in the presence of calcium ions or the adsorption on structural proteins of the collagen type. Therefore, the preparation according to the invention can fulfill functions of thrombocytes.

An in vitro test for the determination of the coagulation time of hemophilia A inhibitor plasma serves for the assessment of the effectiveness of the preparation according to the invention for the treatment of hemophilia A inhibitor patients. An effective preparation shortens the coagulation time by at least 50% and preferably to the coagulation time of normal plasma.

It has been surprisingly demonstrated that, through the association of coagulation factor Xa and phospholipid vesicles, the production of a preparation with good storage stability and a high half-life in a Factor VIII inhibitor plasma is possible. The preparation according to the invention is preferably stored in lyophilized form. The lyophilized preparation can be reconstituted to a solution which comprises the complex in vesicular structure. An addition of carbohydrates such as mono- or disaccharide, approximately in an amount of 3 - 20% (w/w) is advantageous. The stability of the complex according to the invention is further increased in the presence of small amounts of calcium ions. It has been further demonstrated that the complex of coagulation factors and lipid vesicles is not thrombogenic in the pharmaceutical preparations for the treatment of coagulation disorders.

As an active component, the preparation according to the invention comprises a vitamin K dependent protein, for example a factor selected from the group of Factors IIa, VIIa, IXa and Xa, preferably Xa$\beta$. Furthermore, additional proteins can be included, such as for example the Factors II, VII, IX, X, protein C, activated protein C, protein S and protein Z. It has emerged that the combination of Factor Xa with at least one of the named proteins is particularly effective. An additional amount of Factor VIII, activated Factor VIII, and/or vWF, Factor V and/or activated Factor V is advantageous. A further embodiment of the preparation according to the invention comprises a complex of an activated blood coagulation factor and lipid vesicles and additionally " tissue factor" or fragments of "tissue factor".

The preparation according to the invention can simultaneously comprise an agonist and an antagonist. Hence, aside from an active coagulation-promoting substance, an antagonist can also be included in order to alleviate or avoid side effects in the administration of the active coagulation-promoting substance.

The coagulation active substances included in the complex are preferably human and isolated from a plasma fraction or of recombinant origin. Based on a potential infectiousness, a treatment for the inactivation of infectious agents, such as for example viruses or prions, is appropriate. It is recommended to undertake a heat treatment of the substances or the complex of the substances and lipid vesicles. The treatment can ensue for example according to EP 0 159 311.

According to a preferred embodiment, a complex of a highly purified Factor Xa (at least 100 U/mg protein), which is free of infectious agents by a treatment for virus inactivation, with phospholipid vesicles is made available.

Based on its stability, the preparation is to be made available as a liquid preparation without anything further, i.e. without addition of the commonly used stabilizers such as carbohydrates (saccharose) or protease inhibitors (aprotinin).

However, the preparation can in addition also be stored in liquid deep-frozen form or in lyophilized form wherein a polysaccharide amount is advantageous in water removal.

According to the invention, phospholipids in vesicular form can be used as lipid vesicles. The phospholipids can be synthetic or of natural origin. Thereby, a standardized mixture of chemically pure phospholipids is advantageous.

The lipid vesicles can be prepared in various ways such as by sonification of phospholipid dispersions (Barenholz et al., Biochemistry 16, 2806-2810, 1977), the reversed phase evaporation technique (F. Szoka and D. Paphadjopoulos, Proc. Natl. Acad. Sci. USA 75, 4194-4198, 1978), the ethanol injection method (S. Batzri and E.D. Korn, Biochem. Biophys. Acta 298, 1015-1019, 1973) or also the removal of detergents from mixed micelles by dialysis methods (O. Zumbuehl and H.G. Weder, Biochem. Biophys. Acta 640, 252-262, 1981). Furthermore, the extrusion method according to Olson et al. (Biochem. Biophys. Acta 557, 9-23, 1979) can also be used. A dispersion of multilamellar vesicles is prepared by hydration of a lipid film which can be obtained preferably by evaporation of solutions of the lipids in organic solvents with the aid of a rotary evaporator. This dispersion is subsequently pressed through two stacked polycarbonate filters with nitrogen pressure.

This method should be worked at a temperature at which the phospholipids are found in a liquid-crystalline state (at least 5°C, preferably at least 10°C above the phase transition temperature, $T_M$). Optionally, a repeated freeze-thaw cycle is introduced before the extrusion (M.J. Hope et al., Biochem. Biophys. Acta 812, 55-65, 1985). Furthermore, an embodiment has proven to be particularly advantageous. Here, the dispersion of multilamellar vesicles is produced as outlined above, lyophilized, and reconstituted again with water. Thereby, the subsequent extrusion can be carried out particularly easily and also with very high lipid concentrations (for example, 100 mg lipid/ml).

For the production of the complex according to the invention, the described lyophilization step can be carried out after mixing of the vesicle dispersion with the protein, here preferably coagulation factor Xa. After reconstitution, the complex according to the invention can be further extruded. A further possibility for the production of the complex according to the invention is the mixing of the components in the presence of a tenside and the subsequent removal of the tenside, for example by dialysis. A direct hydration of the lipid film with a solution of proteins is also particularly advantageous, wherein optionally lyophilization and reconstitution can be done, whereby the extrusion is made easier. Optionally, in the last variation, the additional lyophilization step can be dispensed with. A direct mixing of the prepared vesicles with the protein in the presence of charged particles or under conditions which permit the incorporation of the protein in and on the lipid vesicles (concentration, selection of proteins with hydrophobic properties, etc.) is also conceivable.

For the extrusion process, particularly filters with a diameter of about 30 to 1000 nm can be used. The typical size of the lipid vesicles (determined by the method of dynamic light scattering) is in the order of 30 to 900 nm, preferably 100 to 500 nm.

The stability of the produced complex is improved when charged particles, for example calcium ions, preferably 1-10 mM, are present. Based on the binding of the coagulation proteins in and on the lipid vesicles, a purification and isolation of the complex per se is possible. This can occur by gel filtration or ultracentrifugation.

The glycerophospholipids phosphatidyl serine, phosphatidic acid, phosphatidyl glycerol, diphosphatidyl glycerol (cardiolipin) and also phosphatidyl ethanolamine which can exhibit a negative charge are among the suitable phospholipids and are used preferably alone or also combined in a mixture with neutral phospholipids such as phosphatidyl choline and sphingomyelin, wherein the binding by addition of calcium ions is favored. As anionic components, the use of other lipids, such as for example sulfatides or also dicetylphosphate, phosphatidyl methanol, phosphatidyl-$\beta$-lactate and oleic acid is also possible. However, the exclusive use of neutral phospholipids such as phosphatidyl choline or sphingomyelin is also conceivable. However, substances obtained through chemical modification such as the lipid derivatives of the polyethylene glycols or substances from the classes of lysophospholipids or glycolipids are also useable. The use of lipids which can exhibit a positive charge, such as for example stearylamine, dimethyl-dioctadecyl ammonium bromide (DDAB), or also cationic lipids of the type of the 1,2-diacyl-3-trimethyl ammonium propanes (TAP) or the 1,2-diacyl-3-dimethyl ammonium propanes (DAP) is also conceivable.

A preferred embodiment comprises the use of lipids with anti-viral effect, for example alkylphospholipids (see EP 0 506 704 B1).

For stabilization, the phospholipid vesicles can comprise up to 80 Mol% cholesterol as an additional component. It is to be emphasized that all specified lipids can also be used as a sole component, optionally also under addition of cholesterol.

For the formation of the complex the lipid vesicles can be found in the gel state or in the liquid-crystalline state, however, the use of systems in the liquid-crystalline state is more favorable. In a version of the invention, lipid vesicles comprised of 1,2,-dioleoyl-sn-glycero-3-phosphocholine and 1-palmitoyl-2-oleoyl-sn-glycero-3-phosphoserine are used (for example in a ratio of PC/PS = 80/20 (w/w), see Example 1) which are liquid-crystalline at room temperature. The use of phospholipids with unsaturated side chains can, however, lead to a reduced stability by virtue of oxidation. Therefore, for the chemical stability of the vesicles, the use of lipids with fatty acid groups such as those of myristic acid, palmitic acid (Examples 2 and 3) or stearic acid, which are however normally found in the gel state, is preferable. The use of these lipids has been proven as particularly advantageous, however, in mixtures with cholesterol.

The invention is more closely illustrated through the following Examples.

4

**Production of phospholipid vesicles**

**Example 1: Production of multilamellar vesicles**

In a 100 ml flask, 40.0 mg 1,2-dioleoyl-sn-glycero-3-phosphocholine and 10.0 mg 1-palmitoyl-2-oleoyl-sn-glycero-3-phosphoserine (Avanti Polar Lipids) are dissolved in 5 ml chloroform and evaporated with the aid of a rotary evaporator under reduced pressure and a temperature of 30°C. After complete removal of the solvent, a vacuum was still held over 30 min at 30 mbar and subsequently dried over a time period of six hours in high-vacuum at 0.1 mbar. The phospholipid film was subsequently hydrated by addition of 5 ml of buffer (20 mM Tris, 150 mM NaCl, pH 7.4) and gentle shaking over one hour at room temperature.

**Example 2**

After formation of a dispersion of multilamellar vesicles according to Example 1, this was extruded 10 times with $N_2$ pressure through two stacked 100 nm polycarbonate filters (10 ml thermobarrel extruder, Lipex Biomembranes Inc., Vancouver, Canada). The determination of the particle size of the vesicles produced in this way with the aid of dynamic light scattering (Malvern Zetasizer 4) resulted in a average diameter of about 100 nm.

**Example 3**

From 35.0 mg 1,2-dimyristoyl-sn-glycero-3-phosphocholine (Nattermann Phospholipid GmbH) and 15.0 mg 1,2-dimyristoyl-sn-glycero-3-phosphoserine (Avanti Polar Lipids) in 5 ml chloroform/methanol mixture (2:1, v/v), a phospholipid film was produced as described in Example 1. After addition of 5 ml of buffer (20 mM Tris, 150 mM NaCl, pH 7.4), the film was hydrated at 50°C by using a rotary evaporator and extruded at 50°C as described under Example 2.

**Example 4**

From a mixture of 35.0 mg 1,2-dipalmitoyl-sn-glycero-3-phosphocholine (Nattermann Phospholipid GmbH) and 15.0 mg 1,2-dimyristoyl-sn-glycero-3-phosphoserine (Avanti Polar Lipids), phospholipid vesicles were produced as described in Example 3. The hydration of the film and the extrusion also occurred at 50°C.

**Example 5**

From a mixture of 40.0 mg 1,2-dioleoyl-sn-glycero-3-phosphocholine (Sigma) and 10.0 mg 1,2-dioleoyl-sn-glycero-3-phosphoglycerol or 17.5 mg cardiolipin from bovine heart (Sigma), phospholipid vesicles were produced as described under Example 1 and 2.

**Example 6**

From a mixture of 40.0 mg 1,2-dioleoyl-sn-glycero-3-phosphocholine and 10.0 mg 1,2-dioleoyl-sn-glycero-3-phosphate (Sigma), phospholipid vesicles were produced as described under Example 1 and 2.

**Example 7**

From a mixture of 35.0 mg 1,2-dioleoyl-sn-glycero-3-phosphocholine, 10.0 mg 1-palmitoyl-2-oleoyl-sn-glycero-3-phosphoserine (Avanti Polar Lipids) and 5 mg phosphatidyl inositol form Soya beans (Sigma), phospholipid vesicles were produced as described under Example 1 and 2.

**Example 8**

5 to 80 Mol% cholesterol (Sigma) was added to the mixtures of phosphatidyl choline, phosphatidyl serine, phosphatidyl glycerol, phosphatidic acid and/or phosphatidyl inositol described in Examples 1 to 7 and extruded as described under the respective Examples.

**Example 9**

After hydration of the phospholipid film, the dispersions from Examples 1-8 were shock frozen (liquid $N_2$) and thawed at 37°C (Examples 2, 5-8) and/or 50°C (Examples 3 and 4). This process was repeated four times. The multilamellar vesicles produced in this way were extruded as described in the respective Examples. The vesicle preparations resulting therefrom had an average diameter of 100 nm.

**Example 10**

As described in the Examples 1-9, multilamellar vesicles were produced through hydration of phospholipid films. The dispersions obtained in this way were frozen at -80°C and lyophilized. After reconstitution of the lyophilizates with the corresponding volumes of $H_2O$, extrusion was done as described under the respective Examples.

**Example 11**

The production of phospholipid vesicles according to Examples 1-10 was carried out in 150 mM NaCl instead of Tris buffer. The size determination of the vesicles with the aid of dynamic light scattering resulted in a nearly identical size distribution in comparison to vesicles that were produced with buffered solution.

**Example 12: 1,2-dioleoyl-sn-glycero-3-phosphocholine**

A mixture of 8.0 mg 1,2-dioleoyl-sn-glycero-3-phosphocholine (Avanti Polar Lipids), 2.0 mg phosphatidyl inositol from Soya beans (Sigma) and 10.0 mg sodium cholate (Sigma) was added in a 50 ml round flask and dissolved in 10 ml of a chloroform/methanol mixture (1:1, v/v). Then, this was evaporated in a rotary evaporator at a bath temperature of 20°C under reduced pressure until dry, taken up in 10 ml absolute methanol and evaporated again. The film produced in this way was taken up under shaking with 2 ml buffer (20 mM Tris, 150 mM NaCl, pH 7.4). For the production of the vesicles, the clear solution was subsequently dialyzed 24 h against the same buffer at 4°C. The determination of the particle size with the aid of dynamic light scattering resulted in an average diameter of about 70 nm.

**Lyophilization of phospholipid vesicles in the presence of saccharose**

**Example 13**

Saccharose to a concentration of 3 - 20% (w/v) was added to the vesicles produced corresponding to the Examples 1-12. Subsequently, this was frozen at -80°C and lyophilized. After reconstitution of the lyophilizate, the determination of the size with the aid of dynamic light scattering resulted in an average diameter of about 100 nm. The electron microscopic examination of this solution resulted in a size distribution of about 80 to 130 nm according to visualization with the negative staining method.

**Production of a complex of Factor Xa and phospholipid vesicles**

**Example 14: colyophilization**

Factor Xa was produced as follows:
A solution of the prothrombin complex preparation (corresponding to 50,000 U Factor X/l) produced according to the methods of Brummelhuis (Methods of Plasma Protein Fractionation, J.M. Curling (editor), page 117 pp., Acad. Press., 1980) and heat treated according to the method of EP 0 159 311, was treated in the presence of 12 % (v/v) Tween®80 one hour at pH 7.0 and room temperature. Subsequently, this was diluted, mixed with trisodium citrate dihydrate (7.0 g/l), and the proteins of the prothrombin complex precipitated at pH 7.0 by addition of 400 ml 1 M barium chloride solution. The precipitate was washed and resuspended in a 25 % (w/v) ammonium sulfate solution containing 50 mM benzamidine HCl. The non-dissolved matter was separated and the solution was brought to 80% saturation with ammonium sulfate in order to precipitate the proteins again. The precipitate was dissolved in buffer and rebuffered chromatogaphically over Sephadex®-G25 against 25 mM trisodium citrate buffer containing 100 mM NaCl and 1 mM benzamidine HCl, pH 6.0.

The protein containing fractions were further purified by means of a DEAE Sepharose® fast flow column. By increasing the sodium chloride concentration in the citrate buffer (25 mM, pH 6.0), Factor X was eluted separated from the other prothrombin complex proteins. The obtained fraction was rebuffered against 20 mM Tris HCl buffer containing 150 mM sodium chloride and 2 mM calcium chloride (pH 7.4). Subsequently, this was mixed with 0.14 mg RVV (Russel's viper venom, a protease from Vipera russellii, Pentapharm) per 100 U FX and stirred one hour at room temperature. The generated Factor Xa was then purified chromatographically with a benzamidine Sepharose® column and concentrated through ammonium sulfate precipitation and subsequently chromatographed over Sephadex®-G25 for the removal of the salt. The produced Factor Xa preparation had a specific activity of at least 100 U Factor Xa/mg protein.

For the production of the complex, a vesicle preparation produced according to the Examples 1-12 was mixed, with or without 2.5 mM calcium chloride, to the Factor Xa fraction. The solution was lyophilized.

After reconstitution, the effectiveness of the preparation in the FEIBA test (test description see AT 350 726) was tested. As a comparison for this, the FEIB activity of the non-complexed Factor Xa was determined. Before the lyophilization, the FEIB activity amounted to 1700 U/ml in both cases. After the lyophilization and reconstitution, 1650 U/ml was recovered in the case of the Factor Xa/PCPS complex. The loss of FEIB activity was much larger in the case of non-complexed Factor Xa. Only 1220 U/ml was recovered.

**Example 15: Extrusion of a colyophilizate**

A colyophilizate produced according to Example 14 comprising 1000 U Factor Xa as well as 50.0 mg of a phospholipid preparation produced according to Example 6 of 40.0 mg 1,2-dioleoyl-sn-glycero-3-phosphocholine and 10.0 mg 1,2-dioleoyl-sn-glycero-3-phosphate was taken up with water in the original volume and extruded twice at a temperature of 20°C through two stacked 400 nm polycarbonate filters (Lipex Biomembranes, Inc.). The determination of the particle size with the aid of dynamic light scattering (Malvern Zetasizer 4) resulted in an average diameter of about 450 nm.

**Example 16: coextrusion**

As described under Example 1, a film was produced from a phospholipid mixture. This was hydrated with a Factor Xa containing solution from Example 14 with or without 2.5 mM calcium chloride. After hydration of the film, it was proceeded as in the Examples 2, 9 and 10 and extruded. Thereby, Factor Xa was complexed with phospholipid vesicles. Saccharose in a concentration of 3 - 20 % (w/v) could be added to the complex produced in this way in order to subsequently lyophilize.

**Production of a complex of activated protein C, Factor Xa and phospholipid vesicles**

**Example 17**

A film was prepared as described under Example 1 from a phospholipid mixture. This was hydrated with the Factor Xa containing fraction from Example 14 as well as with activated protein C in a 20 mM Tris HCl buffer containing 150 mM NaCl and 5 mM $CaCl_2$, pH 7.4. The mixture containing 10 U FXa/ml, 10 U APC/ml and 10 mg phospholipid/ml was lyophilized.

**Purification of the complex of Factor Xa and PCPS vesicles**

**Example 18**

A preparation containing Factor Xa and PCPS vesicles was produced according to the method described in the Examples 14 or 16, wherein saccharose in a final concentration of 5 % (w/v) was added to the Factor Xa phospholipid preparation. The lyophilizate was dissolved in water in such a way that the prepared solution contained 2.4 U Factor Xa/ml and 1 mg/ml PCPS vesicles. A column packed with Superose 6 HR 10/30, Pharmacia, was equilibrated with a buffer (20 mM Tris HCl containing 150 mM NaCl, 0.1 % albumin, 0.01 % Tween 20, 1 mM $CaCl_2$, pH 7.4). 500 $\mu$l of the PCPS vesicles and Factor Xa containing preparation were chromatographed over the column with a flow rate of 0.4 ml/minute.

In the eluate stream, the absorption at 254 nm was measured. The fraction of the exclusion volume containing a complex of PCPS vesicles and Factor Xa was collected.

**Example 19**

A lyophilizate produced according to Example 14 was reconstituted with water such that the finished solution contained 5 U Factor Xa and 5 mg PCPS per ml. 0.5 ml of this solution were mixed with 1 ml of a 30 % (w/v) ficoll 400 solution (Pharmacia, in 150 mM sodium chloride solution). This mixture was placed in an ultracentrifugation tube and overlaid with 3 ml of 10 % ficoll solution. Finally, this was overlaid with 150 ml NaCl solution and subsequently centrifuged by use of a swing out rotor for 30 minutes at 100,000 g and room temperature.

The layer containing the purified Factor Xa/PCPS complex with lower density than the aqueous solution could be separated as a supernatant.

**Example 20**

A complex comprising activated protein C, Factor Xa and phospholipid vesicles was produced according to Example 17. The lyophilized complex was reconstituted with water and concentrated to a third of the starting volume by centrifugation over ultrafree-MC filter units, exclusion limit 100,000 Daltons (polysulphone membranes, Millipore), 30 minutes at 4000 rpm. The retained material obtained in this way contained the purified complex of activated protein C, Factor Xa and phospholipid vesicles.

**Stability of the complex of protein and phospholipids**

**Example 21: Stability of a Factor Xa/PCPS vesicle complex in the lyophilization process**

PCPS vesicles were produced as described in Examples 1 and 2. These were subsequently mixed with Factor Xa, which was produced as described in Example 14, such that the preparation comprised 0.5 mg phospholipid vesicles per ml and 47 U Factor Xa in an aqueous solution of 150 mM/l NaCl and 5 mM/l $CaCl_2$. The complex was lyophilized. As a comparison for this, Factor Xa was lyophilized in the same concentration, however without PCPS vesicles. Subsequently, according to German patent application P4325872.7, the Factor Xa activity was determined in the lyophilized preparations after reconstitution with distilled water in the starting volumes and compared with the Factor Xa amount before the lyophilization (see Table).

| | Factor Xa activity (%) | |
|---|---|---|
| | before lyo | after lyo |
| PCPS/ FXa-complex | 100 | 51 |
| Factor Xa | 100 | 20 |

The Table shows the stabilizing influence of vesicular phospholipid on Factor Xa in the copreparation.

**Example 22: Stability of a Factor Xa/PCPS vesicle complex in solution**

A complex of PCPS vesicles and Factor Xa was produced as described in Example 21 and lyophilized. After the reconstitution, the solution was stored at 22 °C over 20 hours. Subsequently, the activity of Factor Xa was determined. The following Table shows the stability of Factor Xa in the PCPS vesicle complex in comparison to non-complexed Factor Xa.

| | Factor Xa activity (%) | |
|---|---|---|
| | 0 h | 20 h |
| PCPS/ FXa-complex | 100 | 67 |
| Factor Xa | 100 | 34 |

**Example 23: Stability of a protein C/Factor Xa/PCPS vesicle complex in the lyophilization**

A complex comprising protein C, Factor Xa and PCPS vesicles was produced analogously to Example 17 and lyophilized. The preparation contained 0.5 mg phospholipid vesicles/ml, 10 U protein C/ml and 47 U Factor Xa/ml in an aqueous solution of 150 mM NaCl. As a comparison for this, protein C and Factor Xa was lyophilized at the same concentrations, however without PCPS vesicles. Subsequently, the Factor Xa activity was determined as in Example 21 and the protein C activity was determined by use of a chromogenic test (Immunochrom PC, Immuno) in the lyophilized preparations after reconstitution with distilled water in the starting volumes and compared with the respective amount of Factor Xa and protein C before the lyophilization (see Table).

|  | protein C activity (%) | | Factor Xa activity (%) | |
| --- | --- | --- | --- | --- |
|  | before lyo | after lyo | before lyo | after lyo |
| PCPS/protein C/FXa complex | 100 | 89 | 100 | 84 |
| protein C | 100 | 41 | - | - |
| Factor Xa | - | - | 100 | 23 |

The Table shows the stabilizing influence of phospholipid vesicles on protein C and Factor Xa in the complex.

**Example 24: Stability of a protein C/FXa/PCPS vesicle complex in solution**

A complex of protein C, Factor Xa and PCPS vesicles and Factor Xa was produced as described in Example 23 and lyophilized. After the reconstitution, the solution was stored at 22°C over 20 hours. Subsequently, the activity of protein C and Factor Xa was determined. The following Table shows the increased stability of protein C and Factor Xa in the PCPS vesicle complex in comparison to the non-complexed factors.

|  | protein C activity (%) | | Factor Xa activity (%) | |
| --- | --- | --- | --- | --- |
|  | 0 h | 20 h | 0 h | 20 h |
| PCPS/protein C/FXa-complex | 100 | 100 | 100 | 88 |
| protein C | 100 | 65 | - | - |
| Factor Xa | - | - | 100 | 9 |

**In vitro characterization of the protein/phospholipid complexes.**

**Example 25**

For the testing of the effectiveness of the preparation according to the invention, the following test system was used.

100 $\mu$l of FVIII inhibitor plasma (45 Bethesda Units/ml) were mixed in a coagulometer tube with 100 $\mu$l 20 mM Tris HCl buffer containing 150 mM NaCl, pH 7.4 (TBS) and recalcified with a further 100 $\mu$l of a 0.025 M calcium chloride solution. Immediately after addition of the sample to be analyzed (100 $\mu$l), the coagulation time of the mixture was determined by use of a coagulometer (Schnitger/Gross) at 37°C.

When pure TBS buffer was employed as a sample in the represented test substance, the coagulation time amounted to 820 seconds. In comparison to this, the coagulation time of normal plasma in this test system was about 350 seconds.

A preparation produced according to Example 16 was tested at a concentration of 0.88 mU Factor Xa and 0.3 $\mu$g phospholipid/ml. As an additive, either 0.1 U FEIBA produced according to the method of AT 368 883 or 0.1 U recombinant Factor VIIa (Novo Nordisk) were tested. Also, a mixture of the FEIBA preparation (0.01; 0.1; 1.0 U/ml) or of Factor X (0.01; 0.1; 1.0 U/ml) produced according to the method from Example 14 with 0.3 μg/ml phospholipid vesicles produced according to the method from Example 1 and 2 were tested. The influence of the tested substances on the coagulation time is given in the following Table.

Shortening of the coagulation time by a preparation containing the complex of coagulation protein and phospholipid vesicles.

| test substance | concentration (mU/ml) | | | | coagulation time (s) |
|---|---|---|---|---|---|
| | FXa | FEIBA | FVIIa | FX | |
| Factor Xa/PCPS | 0.88 | - | - | - | 125 |
| Factor Xa/FEIBA/PCPS | 0.88 | 100 | - | - | 82 |
| Factor Xa/Factor VIIa/PCPS | 0.88 | - | 100 | - | 131 |
| FEIBA/PCPS | - | 10<br>100<br>1000 | - | - | 409<br>167<br>120 |
| Factor X/PCPS | - | - | - | 10<br>100<br>1000 | 814<br>425<br>336 |

**Biological in vitro activity of various phospholipid vesicle types**

**Example 26**

Phospholipid vesicles comprising mixtures of various phospholipid types in different composition were produced as described in the Examples and processed to copreparations with Factor Xa. The FXa phospholipid vesicle complex resulting therefrom was examined as described in Example 25 on the coagulation shortening effect of FVIII inhibitor plasma. The results (coagulation times) are to be taken from the following Table:

EP 0 680 763 A2

| vesicle type | composition w/w % | conc./ml in test substance | | coagulation time (s) |
| --- | --- | --- | --- | --- |
| | | mU FXa | µg lipid | |
| DOPC POPS | 80 20 | 1.375 | 6.25 | 62 |
| DOPC POPS CHOL | 70 20 10 | 1.375 | 6.25 | 65 |
| DOPC POPS CHOL | 60 20 20 | 1.375 | 6.25 | 57 |
| DOPC POPS CHOL | 50 20 30 | 1.375 | 6.25 | 56 |
| DOPC POPS CHOL | 40 20 40 | 1.375 | 6.25 | 69 |
| DOPC POPS CHOL | 30 20 50 | 1.375 | 6.25 | 58 |
| POPS PI | 95 5 | 1.375 | 6.25 | 73 |
| POPS PI | 90 10 | 1.375 | 6.25 | 77 |
| POPS PI | 80 20 | 1.375 | 6.25 | 71 |
| DOPC PI | 80 20 | 1.375 | 6.25 | 135 |
| DOPC POPS PI | 80 15 5 | 1.375 | 6.25 | 66 |
| DOPC POPS PI | 80 10 10 | 1.375 | 6.25 | 89 |
| DOPC POPS PI | 80 5 15 | 1.375 | 6.25 | 110 |

| vesicle type | composition w/w % | conc./ml in test substance | | coagulation time (s) |
|---|---|---|---|---|
| | | mU FXa | µg lipid | |
| DOPC<br>POPS<br>PI | 75<br>20<br>5 | 1.375 | 6.25 | 75 |
| DOPC<br>POPS<br>PI | 70<br>20<br>10 | 1.375 | 6.25 | 80 |
| DOPC<br>POPS<br>PI | 60<br>20<br>20 | 1.375 | 6.25 | 72 |
| DOPC<br>POPS<br>PI<br>CHOL | 60<br>10<br>10<br>20 | 1.375 | 6.25 | 85 |
| DOPC<br>POPS<br>PI<br>CHOL | 50<br>20<br>10<br>10 | 1.375 | 6.25 | 82 |
| CLP | 100 | 1.16 | 5.63 | 76 |
| DOPC<br>CLP | 70<br>30 | 1.16 | 6.88 | 69 |
| DMPC<br>DMPS | 70<br>30 | 1.16 | 8.45 | 91 |
| DPPC<br>DMPS | 70<br>30 | 1.16 | 8.45 | 123 |
| DMPC<br>DMPS<br>CHOL | 50<br>30<br>20 | 1.16 | 8.45 | 86 |
| DMPC<br>DMPS<br>CHOL | 30<br>30<br>40 | 1.16 | 8.45 | 67 |
| DPPC<br>DMPS<br>CHOL | 50<br>30<br>20 | 1.16 | 8.45 | 100 |
| DPPC<br>DMPS<br>CHOL | 30<br>30<br>40 | 1.16 | 8.45 | 154 |
| DOPG<br>DOPC | 80<br>20 | 1.25 | 10 | 77 |
| DOPA<br>DOPC | 80<br>20 | 1.25 | 10 | 67 |
| buffer background | | 0 | 0 | 820 |

### Legend:

| | | |
|---|---|---|
| DOPC | = | 1,2-dioleoyl-sn-glycero-3-phosphocholine |
| POPS | = | 1-palmitoyl-2-oleoyl-sn-glycero-3-phosphoserine |
| CHOL | = | cholesterol |
| CLP | = | diphosphatidyl glycerin (cardiolipin) |
| PI | = | phosphatidyl inositol |
| DMPC | = | 1,2-dimyristoyl-sn-glycero-3-phosphocholine |
| DMPS | = | 1,2-dimyristoyl-sn-glycero-3-phosphoserine |
| DPPC | = | 1,2-dipalmitoyl-sn-glycero-3-phosphocholine |
| DOPG | = | 1,2-dioleoyl-sn-glycero-3-phosphoglycerol |
| DOPA | = | 1,2-dioleoyl-sn-glycero-3-phosphoric acid |

**Example 27**

The effectiveness of a complex produced according to Example 14 with 20 mU Factor Xa and 4.96 μg PCPS/ml, and various dilutions thereof, in Factor VIII inhibitor plasma was determined in the following coagulation test immediately after mixing and after an hour incubation at 37°C:

200 μl of sample were mixed in a coagulometer tube with 100 μl 20 mM Tris HCl buffer containing 150 mM NaCl, pH 7.4 (TBS) and recalcified with a further 100 μl of a 0.025 M calcium chloride solution. Immediately after addition of this solution, the coagulation time of the mixture was measured by use of a coagulometer (Schnitger/Gross) at 37°C.

When a 1 + 1 mixture of FVIII inhibitor plasma (45 Bethesda Units/ml) and TBS buffer was employed as a sample in the represented test substance, the coagulation time amounted to more than 500 seconds.

As a comparison, pure Factor Xa produced according to the method from Example 14 was tested. The results are summarized in the following Table.

Coagulation time of Factor Xa/PCPS and Factor Xa before and after incubation with Factor VIII inhibitor plasma

| dilution of the stock solution | coagulation time (s) | | | |
|---|---|---|---|---|
| | Factor Xa | | Factor Xa/PCPS | |
| | 0 h | 1 h | 0 h | 1 h |
| undiluted | 77 | 161 | 35 | 69 |
| 1:2 | 95 | 236 | 48 | 111 |
| 1:4 | 117 | 349 | 65 | 185 |
| 1:8 | 152 | >500 | 88 | 314 |
| 1:16 | 224 | >500 | 145 | 400 |
| 1:32 | 314 | >500 | 200 | >500 |
| 1:64 | 441 | >500 | 296 | >500 |
| 1:128 | >500 | >500 | 402 | >500 |

The complex of Factor Xa/PCPS according to the invention has a higher stability than non-complexed Factor Xa. In low concentrations, Factor Xa/PCPS also leads to a shortening of the coagulation time after one hour incubation in Factor VIII inhibitor plasma, whereas non-complexed Factor Xa had already lost its coagulation time shortening activity.

**In vivo characterization of the protein/phospholipid complexes**

**Example 28: Test on thrombogenicity**

A FEIBA preparation produced according to the method of AT 368 883 was tested for thrombogenicity as a complex with PCPS vesicles and in non-complexed form in the Wessler-Stasis model. The PCPS vesicles were produced according to the method from Example 1 and 2, wherein vesicles of average sizes of 100 nm and/or 50 nm were obtained through a suitable filter choice. 4 U FEIBA and 60 $\mu$g PCPS were used per kg rabbit. The thrombogenicity of the FEIBA preparation was not increased by the complex formation with PCPS vesicles. In all cases, no thrombi formation was registered in the stasis model (score = 0-1).

**Heat treatment of the protein/phospholipid vesicle complexes**

**Example 29: Heat treatment of a complex of Factor Xa and PCPS vesicles**

A lyophilizate of Factor Xa and PCPS produced according to Example 14 or 16 was treated according to the method of EP 159 311 for 10 hours at 60°C and 1 hour at 80°C. The Factor Xa activity of the reconstituted solution amounted to more than 90% of the activity before the heat treatment. With the aid of the method of dynamic light scattering (Malvern Zetasizer 4) the preservation of the vesicular structure could be detected.

**Example 30: Heat treatment of a complex of FEIBA and PCPS vesicles**

A complex of phospholipid vesicles and FEIBA was produced analogously to Example 14 and lyophilized. After reconstitution, the lyophilizate contained 30 U FEIBA/ml and 3.4 mg PCPS/ml in a buffer of 4 g Na$_3$citrate•2H$_2$O/l and 8 g NaCl/l, pH 7.0. A lyophilizate corresponding to this composition was treated according to the method of EP 159 311 for 10 hours at 60°C and 1 hour at 80°C. The FEIBA activity in the reconstituted solution after heat treatment amounted to more than 90% of the activity before the heat treatment With the aid of the method of dynamic light scattering (Malvern Zetasizer 4) the preservation of the vesicular structure could be detected.

## Claims

1. Stable preparation for the prevention and treatment of blood coagulation disorders that comprises an active coagulation-promoting substance which is bound in and/or on lipid vesicles and is suitable for intravenous administration.

2. Preparation according to claim 1, characterized in that the active coagulation-promoting substance is an activated coagulation factor.

3. Preparation according to claim 2, characterized in that the activated coagulation factor is a vitamin K dependent protein.

4. Preparation according to claim 3, characterized in that the activated coagulation factor is selected from the group consisting of Factors IIa, VIIa, IXa and Xa.

5. Preparation according to claim 4, characterized in that Factor Xa$\beta$ is included.

6. Preparation according to any one of claims 1 to 5, characterized in that a protein from the group comprising Factor II, VII, IX, X, protein C, activated protein C, protein S and protein Z is additionally included.

7. Preparation according to any one of claims 1 to 6, characterized in that "tissue factor" or a fragment of "tissue factor" is additionally included.

8. Preparation according to any one of claims 1 to 7, characterized in that Factor VIII, activated Factor VIII and/or von Willebrand Factor is additionally included.

9. Preparation according to any one of claims 1 to 8, characterized in that Factor V and/or activated Factor V is additionally included.

10. Preparation according to claim 1, characterized in that it comprises lipid vesicles for the treatment of thrombocyte-caused blood coagulation disorders which are bound with a thrombocytic coagulation active substance alone or in combination with further coagulation active substances and can fulfill the functions of thrombocytes.

11. Preparation according to one of the above claims, characterized in that it is simultaneously comprises an agonist and an antagonist.

12. Preparation according to one of the above claims, characterized in that the lipid vesicles have a size determined according to the method of dynamic light scattering in the range of 30 to 900 nm, preferably 100 to 500 nm.

13. Preparation according to one of the above claims, characterized in that it does not comprise common stabilizers such as carbohydrates or protease inhibitors.

14. Preparation according to claim 13, characterized in that the preparation is present in the form of an aqueous solution and more than 40% of the activity of the active coagulation-promoting substance remains by storage at 22°C after 20 hours.

15. Preparation according to one of the claims 1 to 12, characterized in that it is present in liquid-frozen or lyophilized form.

16. Preparation according to one of the claims 1 to 15, characterized in that the active coagulation-promoting substance was treated for the inactivation of potentially present viruses.

17. Preparation according to one of the claims 1 to 15, characterized in that the preparation was treated for the inactivation of potentially present viruses.

18. Preparation according to claim 17, characterized in that the preparation is treated by chemical and/or physical measures.

19. Preparation according to claim 18, characterized in that the preparation is heat treated.

20. Preparation according to one of the claims 16 to 18, characterized in that the active coagulation-promoting substance is included with a biological activity of at least 90% with respect to the activity before the inactivation treatment.

21. Method for the production of a preparation according to claim 1, characterized in that a lyophilization step is carried out after mixing a vesicle dispersion with the active coagulation-promoting substance.

22. Method for the production of a preparation according to claim 1, characterized in that a direct hydration of a lipid film occurs with a solution of the active coagulation-promoting substance.

23. Method for the production of a preparation according to claim 1, characterized in that the prepared vesicles are mixed directly with the active coagulation-promoting substance in the presence of charged particles or are mixed under conditions in order to permit an incorporation of the active coagulation-promoting substance in and on the lipid vesicles.

24. Method according to one of the claims 21 to 23, characterized in that the method additionally comprises a step in which the active coagulation-promoting substance bound in and/or on lipid vesicles is subjected to a treatment for virus inactivation.